# EUROPEAN PATENT APPLICATION

(11) **EP 4 353 352 A1**
(43) Date of publication of application: **17.04.2024**
(21) Application number: 22201167.8
(22) Date of filing: 12.10.2022
(51) Int. Cl.: B01J 19/10, B33Y 99/00, B29C 64/10, B29C 64/165, B33Y 10/00, B22F 12/00

(54) **APPARATUS AND METHOD FOR ASSEMBLING PARTICLES IN A WORKING MEDIUM TO AN AGGLOMERATE UNDER THE EFFECT OF ACOUSTIC FORCES**

(71) Applicant: Max-Planck-Gesellschaft zur Förderung der Wissenschaften e.V., 80539 München (DE)
(72) Inventor: Melde, Kai, 69120 Heidelberg (DE); Fischer, Peer, 69120 Heidelberg (DE)
(74) Representative: v. Bezold & Partner Patentanwälte - PartG mbB

(57) **Abstract**

The invention relates to an apparatus (100) for assembling particles (1) in a working medium (2) to an agglomerate (3) under the effect of acoustic forces. The apparatus (100) comprises a first acoustic source device (10) configured for emitting a first acoustic holographic interference field (11) along a first direction (12) and a second acoustic source device (20) configured for emitting a second acoustic holographic interference field (21) along a second direction (22). Further, the first acoustic source device (10) and the second acoustic source device (20) are arranged such that the first acoustic holographic interference field (11) and the second acoustic holographic interference field (21) are superimposed with each other in an assembling portion (101) to form an acoustic pressure field, which defines a shape of the agglomerate (3). Further, the first acoustic source device (10) and the second acoustic source device (20) are arranged such that the first direction (12) and second direction (22) are tilted relative to each other. Furthermore, a method for assembling particles (1) in a working medium (2) to an agglomerate (3) under the effect of acoustic forces is described.

## Description

The invention relates to an apparatus for assembling particles (e.g. biological cells or hydrogel beads) in a working medium (e.g. water) to an agglomerate (e.g. a 3D object). The shape of the agglomerate is obtained by utilizing acoustic forces of an acoustic pressure field, resulting from the superposition of at least two acoustic holographic interference fields. For this, the apparatus comprises at least two acoustic source devices emitting the respective acoustic holographic interference fields. Furthermore, the invention relates to a method for assembling particles in a working medium to an agglomerate under the effect of acoustic forces. The method is contactless and can be, for example, performed with the apparatus mentioned before. Applications of the invention are available in the fields of fabricating materials with arbitrary shapes, additive manufacturing, rapid prototyping, and bio-fabrication. The invention can, for example, be used for assembling cell cultures to construct spheroids, organoids and/or tissues of defined shapes, composition and size. The assembled particles may be organic, inorganic, or any combination thereof.

Conventional methods used for spheroid and organoid fabrication offer very limited control over size and shape. Current solutions, like 3D bioprinting or stereolithography, typically suffer long build times and low cell viability. Further, these techniques are limited in that they do not manipulate the cells directly. Rather, they print inks (so-called bio-inks) or polymers (e.g. hydrogels) that support the growth of cells. Many of those bio-inks further contain the cells mixed into the liquid phase. To print a co-culture of different cell types it is then required to have multiple extruder units each connected to a separate bio-ink reservoir. The maximum achievable cell concentration for these techniques is thus limited for various reasons. First, a too high density and/or a lack of nutrients or dissolved gases will negatively affect cell viability. Second, the shear rates encountered in the extruder nozzle increase with higher cell density. Higher shear rates are known to negatively affect cell viability. High shear rates further cause individual cells stress and might lead to unwanted effects, such as cell death or differentiation of stem cells into the wrong cell type. Typically, the cell density is low in conventional printing methods.

For a better control on the arrangement of material and to achieve higher packing densities, EP 3 034 281 B1 discloses an apparatus for fabricating a component by directly manipulating the material utilizing an acoustic field and the effect of acoustic forces. For this, the apparatus includes an acoustic source device being arranged for creating acoustic forces and positioning a precursor material in a working medium under the effect of the acoustic forces, so that a material distribution is formed, which has a shape of a component to be fabricated. However, in particular, in the case of complex 3D structures, the solution of EP 3 034 281 B1 does not always allow for assembling/positioning the material as precisely as desired.

Accordingly, it is an object of the invention is to provide an improved solution for assembling material via acoustic forces, wherein disadvantages and limitations of conventional techniques are avoided. In particular, the solution should allow for contactless assembling material in arbitrary shapes with high precision.

These objectives are solved by an apparatus and a method, comprising the features of the independent claims, respectively. Preferred embodiments and applications of the invention result from the dependent claims.

According to a first general aspect of the disclosure, an apparatus is provided for assembling particles, preferably microparticles (e.g. biological cells, organoids, and/or gel beads), to an agglomerate under the effect of acoustic forces (e.g. acoustic radiation forces). The agglomerate may have a one-, two-, or three-dimensional shape. The particles may be organic (e.g. comprising bacteria, tissue samples, hydrogels, and/or polymers), inorganic (e.g. comprising polystyrene, glass, ceramics and/or metal), or any combination thereof. Further, the particles may be contained (e.g. suspended) in a working medium (e.g. water or a hydrogel). Preferably, the apparatus may, therefore, be an apparatus for assembling particles in a working medium to an agglomerate under the effect of acoustic forces.

The apparatus comprises a first acoustic source device (e.g. a first ultrasound transducer with a first acoustic hologram) configured for emitting a first acoustic holographic interference field along a first direction. Further, the apparatus comprises a second acoustic source device (e.g. a second ultrasound transducer with a second acoustic hologram) configured for emitting a second acoustic holographic interference field along a second direction. As indicated by the expressions "first" and "second", the first direction and the second direction may be different (differently orientated).

Furthermore, the first acoustic source device and the second acoustic source device are arranged such that the first acoustic holographic interference field and the second acoustic holographic interference field are superimposed with each other in an assembling portion (of the apparatus) to form a, preferably stationary, acoustic pressure field. The acoustic pressure field may be formed by interference of the first and second acoustic holographic interference field in the assembling portion. The acoustic pressure field may have a distribution of acoustic pressure extrema (acoustic pressure maxima and/or acoustic pressure minima). Preferably, the acoustic pressure field defines a shape of the agglomerate. The acoustic pressure field may, therefore, have a configuration/pattern, which may be (set) such that it corresponds to that of the agglomerate to be assembled, which will be outlined in more detail herein.

In the region of the acoustic pressure field, the particles may be moved by the effect of acoustic forces. Depending on the properties (e.g. compressibility, absorptivity, and/or reflectivity) of the particles and/or the working medium, different acoustic forces may act as translation forces onto the particles. As a consequence, each particle (e.g. in a mixture or suspension) may move in a direction, which is determined by a gradient of the energy, and once it has moved to an energetically more favourable location, it can be held in this location or in the neighbourhood thereof. Accordingly, the particles may be moved towards the energy extrema of the acoustic pressure field. Preferably, the particles are collected around energy extrema of the acoustic pressure field (accumulation at the energy extrema) or at a holding surface, which is crossed by the gradients of energy provided by the acoustic pressure field. The holding surface can be a plane or curved surface. As an example, particles with a compressibility, which is lower than the compressibility of the working medium, may be moved to the portions of the acoustic pressure field, which have minimum pressure variations (e.g. pressure nodes). Otherwise, particles with a compressibility, which is larger than the compressibility of the working medium, may be moved to portions of the acoustic pressure field, which have maximum pressure variations. By this, said agglomerate may be formed, wherein its shape is determined by the acoustic pressure field.

In order to advantageously provide precise control on the assembling of the particles, the first acoustic source device and the second acoustic source device are arranged such that the first direction and second direction are tilted relative to each other. Preferably, the first and second direction are, therefore, not parallel and antiparallel orientated. For example, the first and second direction may be orientated perpendicular to each other. By this, the particles can advantageously be targeted from different spatial directions by acoustic forces, creating a kind of contactless "acoustic pliers" or "acoustic tweezers", which allow for a stable and precise arrangement of the particles in the assembling portion. Furthermore, the claimed solution does, advantageously, neither require any superposition of standing waves nor phase discontinuities to trap the particles in 3D. Besides the possibility of assembling arbitrary 3D shapes, the apparatus advantageously provides high flexibility in the experimental setup as the first and second acoustic source devices can be placed on one side of the projected field (e.g. on a hemisphere, which surrounds the assembling portion from below), which leaves room for experimental intervention, i.e. the addition of material, access for optical characterization and/or imaging, and/or the integration with other processes.

In this context, the term "acoustic holographic interference field" may refer to an acoustic field, which is generated by interference of several sub-waves (in a holographic process). The sub-waves may be created e.g. by at least one acoustic diffraction element (e.g. a phase plate or hologram arranged in the acoustic source device) and/or an acoustic array device, i.e. an integrated array of acoustic sources. Consequently, the acoustic holographic interference fields emitted by the first and second acoustic source devices are each preferably not simple plane or spherical wave sound fields, but rather comprise wave fronts with a patterned/structured phase and/or amplitude distribution. In other words, the first/second acoustic holographic interference field may each comprise wave fronts with a (spatially) patterned/structured phase and/or amplitude distribution. Preferably, the pattering/structuring of the phase distribution is such that the respective acoustic holographic interference field has a predefined structure in the assembling portion distant from its acoustic source device, wherein said structure depends on the agglomerate to be assembled.

According to a first aspect of the disclosure, the first direction and the second direction may be tilted (e.g. inclined) relative to each other by an angle in a range from 10° to 170°, preferably in a range from 45° to 135°, particular preferred in a range from 80° to 100°. Thus, the first and second direction may intersect each other at an (intersection) angle in a range from 10° to 170°, preferably in a range from 45° to 135°, particular preferred in a range from 80° to 100°. By this, advantageously, a solid angle of irradiation/insonification of the assembling portion can be increased.

According to a further aspect, the first direction and the second direction may be perpendicular to each other. Consequently, the first and second acoustic source device may be positioned in an orthogonal arrangement. Such an arrangement was advantageously found in particular versatile.

According to a further aspect, the first acoustic source device and the second acoustic source device may be both arranged on a (imaginary) hemisphere around the assembling portion. Preferably, the assembling portion is located in a centre of the hemisphere. Furthermore, the hemisphere may be open to the top and/or the first and second acoustic source device may be located below the assembling portion.

According to a further aspect, the first acoustic source device and the second acoustic source device may not face each other. Preferably, the first and second acoustic source may, therefore, not positioned in a planar and/or standing-wave configuration. Instead, the first and second acoustic source may be positioned such that the first/second acoustic holographic interference field does not interact with the second/first acoustic source device, such that in particular (unwanted) field reflections at the acoustic source devices can be advantageously avoided. The first and second acoustic source device may be positioned such that, each of the first and second acoustic holographic interference field can propagate freely without interacting any of the acoustic source devices.

According to a further aspect, the first acoustic source device and the second acoustic source device may be rotatable about a common rotation axis. Preferably, this common rotation axis may be orientated vertically. For example, the first and second acoustic source device may be fixed to a single mount, which may be rotatable around the common axis. Advantageously, this provides further degree of freedom for assembling, in particular for realizing complex shaped agglomerates.

According to a further aspect, the first acoustic source device may have a curved (e.g. spherical or cylindrical) emitter surface, which may also be referenced as "first emitter surface" in the following. Accordingly, the first acoustic source device may have a non-planar shape. Preferably, said first emitter surface is arranged such that it lies on said hemisphere and/or follows the geometry of said hemisphere. In addition or alternatively, also the second acoustic source device may have a curved (e.g. spherical or cylindrical) emitter surface, which may also be referenced as "second emitter surface" in the following. Accordingly, the second acoustic source device may have a non-planar shape. Preferably, said second emitter surface is arranged such that it lies on said hemisphere and/or follows the geometry of said hemisphere.

According to a further aspect, the first acoustic source device may be a (e.g. frequency-tunable) ultrasound source device. For example, the first acoustic source device may be configured for emitting sound waves in a frequency range from 100 kHz to 50 MHz. Consequently, the first acoustic holographic interference field may have an acoustic holographic interference field frequency in the ultrasound frequency range, e.g. in a frequency range from 100 kHz to 50 MHz. In addition or alternatively, also the second acoustic source device may be a (e.g. frequency-tunable) ultrasound source device. For example, the second acoustic source device may be configured for emitting sound waves in a frequency range from 100 kHz to 50 MHz. Consequently, the second acoustic holographic interference field may have an acoustic holographic interference field frequency in the ultrasound frequency range, e.g. in a frequency range from 100 kHz to 50 MHz.

According to a further aspect, the first acoustic source device and/or the second acoustic source device may be a (e.g. dynamic) phased array transducer, respectively. For example, the phased array transducer may comprise an (e.g. integrated) array of acoustic sources (e.g. piezoelectric oscillators), which are individually controllable and each of which being adapted for emitting an acoustic wave. By individually controlling the respective acoustic sources of the array sub-waves may be emitted, which are superimposed (within the phased array transducer) and interfere with each other for generating the first/second acoustic holographic interference field. Advantageously, the use of phased array transducers allows for flexible creating different shapes of agglomerate, without the necessity of hardware changes in the apparatus.

According to a further aspect, the first acoustic source device may comprise a (e.g. single) first acoustic source, like for example a piezoelectric oscillator, and/or a first acoustic diffractive element. In addition or alternatively, also the second acoustic source device may comprise a (e.g. single) second acoustic source, like for example a piezoelectric oscillator, and/or a second acoustic diffractive element. In this context, the term (first/second) "acoustic diffractive element" may refer to a material, which is capable of interacting with a wave-front of a primary acoustic wave such that the phases and/or amplitudes across the wave-front are changed by diffraction in a predetermined way. In detail, upon incident of the primary acoustic wave (e.g. a plane or spherical primary acoustic wave) on the acoustic diffractive element, the acoustic diffractive element may generate several acoustic sub-waves interfering with each other. Thereby, each changed phase and/or amplitude may provide a sub-wave contribution for the subsequent interference. Consequently, in this case, the first/second acoustic holographic interference field may be generated via self-interference of the primary acoustic wave emitted from the (single) acoustic source of the first/second acoustic source device. For example, the first/second acoustic diffractive element may be a (e.g. static) acoustic hologram (e.g. a holographic phase mask), a (e.g. dynamic) spatial acoustic modulator (e.g. a microbubble array), and/or an acoustic (e.g. labyrinthine) metamaterial. The structure of the first/second acoustic diffractive element for generating the (desired) first/second acoustic holographic interference field may be calculated using an algorithm for computer-based calculation of holograms, as it is known from optics, e.g. with the Gerchberg-Saxton-Algorithm. The calculation can readily be adapted to acoustics. The use of acoustic diffractive elements has particular advantages with regard to a simplified structure of the apparatus.

According to a further aspect, the apparatus may further comprise at least one further acoustic source device (e.g. a third, fourth, fifth acoustic source device etc.) configured for emitting at least one further acoustic holographic interference field along at least one further direction. The at least one further direction may be different (differently orientated) to each of the first and second direction. Further, the at least one further acoustic source device may comprise features mentioned in the context of the first/second acoustic source device (e.g. ultrasound source device, arranged on the hemisphere, curved emitter surface, etc.), wherein, to avoid repetition, reference is only made to the relevant sections, without explicitly listing them here. The at least one further acoustic source device may be arranged such that the at least one further acoustic holographic interference field is superimposed with the first and second acoustic holographic interference fields in the assembling portion for further forming the acoustic pressure field. Thereby, the at least one further acoustic source device is arranged such that the at least one further direction is tilted (e.g. perpendicular) with respect to the first direction and/or second direction. In a preferred embodiment, all acoustic source devices may be positioned in an orthogonal arrangement, such that the respective (emission) directions are all perpendicular to each other. Advantageously, by using multiple acoustic source devices, the particles can be targeted from multiple different spatial directions by acoustic forces, creating a kind of contactless multidimensional "acoustic pliers" or "acoustic tweezers". This advantageously allows for realizing more complex shaped agglomerates.

According to a further aspect, the apparatus may further comprise a reservoir (e.g. open at the top) designed to contain a liquid (e.g. water). For example, the reservoir may be a basin and/or tank. Preferably, the reservoir is a liquid-filled reservoir, i.e. filled with the liquid. The first acoustic source device and the second acoustic source device (and preferably the at least one further acoustic source device) may be arranged at the reservoir. For example, the first and second acoustic source device may be arranged at least partially in the reservoir or surrounding the reservoir. In addition or alternatively, the first and second acoustic source device may be attached to a wall (e.g. a side-wall and/or bottom-wall) of the reservoir. Preferably, the first and second acoustic source device may be arranged for acoustically coupling sound in the liquid, when the container is filled with the liquid.

According to a further aspect, the apparatus may further comprise a (e.g. open at the top) reservoir insert designed to contain a further liquid (e.g. sterile water). The reservoir insert may at least partially be inserted in the reservoir to divide the reservoir into two fluidically separated portions. For example, the reservoir insert may be arranged to separate the reservoir in an outer portion, which may be located between an outer wall of the reservoir insert and an inner wall of the reservoir, and an inner portion located in the reservoir insert. The reservoir insert may be arranged such that the assembling portion is located in the reservoir insert or in the inner portion. Preferably, the reservoir insert is removable. For example, the reservoir insert may be configured for only being temporally hung and/or mounted in the reservoir (e.g. for the assembling process) and being removed from the reservoir (e.g. for a sterilization/autoclave process of the reservoir insert). Advantageously, by this a sterile environment can be provided, in particular necessary for biological samples.

According to a further aspect, the apparatus may further comprise a flow-through chamber for guiding the particles and/or the working medium. For example, the flow-through chamber may be at least partially arranged within the reservoir. The flow-through chamber may comprise a housing with an inlet, an outlet, and a connection segment, wherein the connection segment connects the inlet and outlet. For example, the connection segment may have a tubular and/or hose-like section. The connection segment may further be configured for directing a flow of the particles and/or the working medium from the inlet to the outlet. Preferably, the assembling portion is arranged within the connection segment. For this, the first and second acoustic source devices may be arranged such that the first and second acoustic holographic interference fields are superimposed within the connection segment. Preferably, the housing of the connection segment may have a thickness sufficiently thin and/or matched with the acoustic wavelength (e.g. a multiple of half the wavelength) to appear sound-transparent for the first and second acoustic holographic interference fields. Advantageously, the flow-through chamber allows for transporting particles and/or the working medium to and/or from the assembling portion. For example, the flow-through chamber may enable a nutrient and/or gas (e.g. CO₂) exchange during the assembling process, which is in particular important in case of biological samples.

According to a further aspect, the connection segment of the flow-through chamber may further comprise a (e.g. semi-permeable) membrane (e.g. a dialysis membrane). For example, the membrane may have a bag-like structure and/or configured as a membrane bag. Preferably, the connection segment of the housing may comprise an opening, to which the membrane (e.g. membrane bag) is fixed, particular preferred such that the membrane protrudes from the opening into the interior of the connection segment (e.g. in a bag-like manner) and/or seals the opening. The membrane may preferably being arranged for forming a flow-calmed portion in the flow-through chamber. Further, the membrane may be arranged to separate the assembling portion from the flow of the particles and/or the working medium. By this, for example, the assembled agglomerate can be trapped at the assembling portion, while nutrients and/or gases can be transported via the working medium to the membrane and pass the membrane to reach the assembling portion. By this, advantageously, the assembling can be performed in a way that decouples the media exchange from the sample addition (assembling).

According to a further aspect, the apparatus may comprise a sample container to contain the particles and/or the working medium. For example, the sample container may be a tube- and/or cuvette-like container. The sample container may be removable. For example, the sample container may be configured for only being temporally inserted in the apparatus (e.g. for the assembling process) and being removed from the apparatus (e.g. for further processing of the agglomerate). Further, the sample container may comprise a wall/housing, wherein a thickness of the wall/housing is sufficiently thin and/or matched with the acoustic wavelength (e.g. a multiple of half the wavelength) to appear sound-transparent for the first and second acoustic holographic interference fields. Preferably, the sample container may be arranged at the assembling portion. For this, the apparatus may comprise a sample container holding device, which is configured to position and/or fix the sample container at the assembling portion. In particular, in case of biological samples/particles, the use of a sample container advantageously allows for providing a sterile environment for the assembling of the particles and/or growth of the agglomerate.

According to a further aspect, the sample container may comprise multiple (e.g. three) compartments. For example, the compartments may be arranged parallel and/or side by side to each other. Preferably, the multiple compartments may be connected in pairs via a fluid connection and/or a semi-permeable membrane. Thereby, the multiple compartments may, for example, be connected in series. Advantageously, this allows for performing individual assemblies in parallel or sequentially, without hardware changes within the apparatus.

According to a further aspect, the sample container may comprise at least one optically transparent wall and/or a removable cover (e.g. a lid).

According to a further aspect, the sample container may comprise a manipulation port made of an elastomeric material for an insertion of needles. Advantageously, this allows for interacting with the particles and/or working medium without opening the sample container on a large scale, such that, a contact of the particles and/or working medium with air can, for example, be effectively avoided.

According to a further aspect, the sample container may comprise a (e.g. needle-like and/or beam-line) resonance element, configured to vibrate when exited by soundwaves. Preferably, the resonance element is arranged inside the sample container. For example, the resonance element may comprise a first end portion, which is fixed to an inner wall of the sample container, and a second free end portion, which lies opposite to said first end portion. The resonance element may be configured for generating a streaming in the sample container, when the sample container is filled with the particles and/or the working medium and when the resonance element is excited with soundwaves (e.g. 100 kHz). Advantageously, this enables quick and efficient mixing of liquids or re-suspension of sedimented particles, without direct access. This way, the sample container can advantageously stay closed and sealed during mixing, an advantage for sterile operation.

According to a further aspect, the sample container may be made of a soft, dissolvable and/or enzymatically digestible material. Further, the sample container may be made of a material that is sound-transparent.

According to a further aspect, the apparatus may further comprise a sample container holding device. The sample container holding device may, thereby, be configured for fixing the sample container in a predefined position. The predefined position may, for example, be a position, where the sample container is positioned at the assembling portion. In addition or alternatively, the sample container holding device may be configured for rotating the sample container about a predefined rotation axis. The predefined rotation axis may be vertical, for example. Advantageously, this provides a further degree of freedom for the assembling of the particles.

According to a further aspect, the apparatus may further comprise a light projecting device configured for illuminating the assembling portion with light (e.g. visible light or ultraviolet light), preferably to locally trigger chemical reactions and/or to induce local solidification of the agglomerate. For example, the light projecting device may comprise a digital micro mirror device and/or a spatial light modulator device. Preferably, the light projecting device is configured for locally illuminating specific regions of the assembling portion and/or for illuminating different regions of the assembling portion differently. In other words, preferably the light projecting device is not configured for uniformly illuminating the whole assembling portion with the same power. Advantageously, this can be used to locally fix particle positions by locally activating binding sites of the particles (e.g. functionalized cells).

According to a further aspect, the apparatus may further comprise a sedimentation-prevention acoustic source device (e.g. an unfocussed ultrasound transducer). The sedimentation-prevention acoustic source device may be arranged (e.g. at the bottom of the apparatus) for emitting soundwaves directed opposite to the direction of gravity, preferably to prevent sedimentation of the particles and/or the agglomerate. The sedimentation-prevention acoustic source device may be configured to emit plane and/or spherical soundwaves, for example. In contrast to the first and second acoustic source devices, which are each configured for emitting a (sophisticated/complex) acoustic holographic interference field, the sedimentation-prevention acoustic source device may be configured as a common (simple) transducer, without the need for shaping the wave fronts in a specific way. The sedimentation-prevention acoustic source device may be frequency-tuneable for enabling a (particle) size dependent sedimentation-prevention. For example, an unfocused wave of 3 MHz, 400 kPa may be used to prevent sedimentation of larger aggregates without affecting single particles (e.g. cells). The frequency may also be continuously lowered while an aggregate forms to always provide the optimal levitation force to counteract sedimentation. In contrast, the first and second acoustic source devices (used for assembling the particles) may perform at a higher frequency (e.g. 10 MHz).

For the skilled person it is immediately apparent that the afore-mentioned aspects (reservoir, flow-through chamber, sample container, sedimentation-prevention acoustic source device etc.) described in connection with the use of two acoustic source devices (first and second acoustic source devices), may also be realized with only one acoustic source device. Consequently, these aspects are also disclosed and claimable independent of the presence of the further (second) acoustic source device. In detail, the afore-mentioned aspects (technically feasible without the second acoustic source device) are also disclosed (individually or in combination) in connection with an apparatus for assembling particles (e.g. in a working medium) to an agglomerate under the effect of acoustic forces, comprising: at least one acoustic source device configured for emitting an acoustic holographic interference field (e.g. along a predefined direction), wherein the at least one acoustic source device is arranged such that the acoustic holographic interference field forms an acoustic pressure field in an assembling portion, wherein the acoustic pressure field defines a shape of the agglomerate.

According to a second general aspect of the disclosure, a method is provided for assembling particles, preferably microparticles (e.g. biological cells, organoids, and/or gel beads), to an agglomerate under the effect of acoustic forces (e.g. acoustic radiation forces). The agglomerate may have a one-, two-, or three-dimensional shape. The particles may be organic (e.g. comprising bacteria, tissue samples, hydrogels, and/or polymers), inorganic (e.g. comprising polystyrene, glass, ceramics and/or metal), or any combination thereof. Further, the particles may be contained (e.g. suspended) in a working medium (e.g. water or a hydrogel). Preferably, the method may, therefore, be a method for assembling particles in a working medium to an agglomerate under the effect of acoustic forces.

Preferably, the method is performed by using an apparatus described herein. Accordingly, the features described in connection with the device shall also be disclosed and claimable in connection with the method. The same applies vice versa.

The method comprises emitting a first acoustic holographic interference field along a first direction (e.g. by a first acoustic source device) and emitting a second acoustic holographic interference field along a second direction (e.g. by a second acoustic source device). Preferably, the first and second acoustic holographic interference fields are emitted with the same frequency (e.g. a frequency in a non-audible frequency range, like an ultrasound frequency, for example). Particularly preferred, the first and second acoustic holographic interference fields have a frequency of at least 20 kHz, in particular at least 40 kHz, up to the GHz-range. In a further preferred embodiment, the first and second acoustic holographic interference fields are emitted synchronized to each other. For example, should there be no (global) phase shift in the control and/or excitation of the corresponding fields.

Furthermore, the first direction and the second direction are tilted (e.g. inclined) relative to each other. For example, the first direction and the second direction may be tilted relative to each other by an angle in a range from 10° to 170°, preferably in a range from 45° to 135°, particular preferred in a range from 80° to 100°. In a particular preferred embodiment, the first and second direction may be orientated perpendicular to each other.

The method further comprises superimposing the first acoustic holographic interference field and the second acoustic holographic interference field with each other in an assembling portion to form a, preferably stationary, acoustic pressure field. The step of superimposing may thereby comprise interfering of the first and second acoustic holographic interference field in the assembling portion. The resulting acoustic pressure field may have a distribution of acoustic pressure extrema (acoustic pressure maxima and/or acoustic pressure minima). Preferably, the acoustic pressure field defines a shape of the agglomerate. The acoustic pressure field may, therefore, have a configuration/pattern, which is (set) such (e.g. calculated based on the Gerchberg-Saxton-Algorithm) that it corresponds to that of the agglomerate to be assembled.

The method further comprises providing the particles in the assembling portion. This can, for example, comprise positioning a sample container comprising the particles and/or the working medium at the assembling portion. In addition or alternatively, the step of providing the particles may comprise, for example, transporting the particles and/or the working medium to the assembling portion via a flow-through chamber.

The method further comprises assembling the particles to the agglomerate in the assembling portion via the acoustic pressure field. The step of assembling may, therefore, comprise moving the particles by the effect of acoustic forces of the acoustic pressure field. As outlined in detail before, depending on the properties (e.g. compressibility, absorptivity, and/or reflectivity) of the particles and/or the working medium different acoustic forces may act as translation forces onto the particles. As a consequence, each particle (e.g. in a mixture or suspension) may move in a direction, which is determined by a gradient of the energy, and once it has moved to an energetically more favourable location, it can be held in this location or in the neighbourhood thereof. Accordingly, the step of assembling may comprise moving the particles towards energy extrema of the acoustic pressure field or to a holding surface, which is crossed by the gradients of energy provided by the acoustic pressure field.

As outlined in the context of the apparatus, also by said method the particles can advantageously be targeted from different spatial directions by acoustic forces, creating a kind of contactless "acoustic pliers" or "acoustic tweezers", which allow for a stable and precise arrangement of the particles in the assembling portion. Furthermore, the method does advantageously neither require any superposition of standing waves nor phase discontinuities to trap the particles in 3D.

According to an aspect, the particles may comprise at least two different types of particles. For example, the particles may comprise particles of type A (e.g. biological cells) and particles of type B (e.g. gel beads), which have different properties. Preferably, the at least two different types of particles may be selected from the following materials: a biological cell, a stem cell, a spheroid, an organoid, a tissue fragment, a biomolecule, a bacterium, a virus, a gel bead, a vesicle, a tissue scaffold material, a hydrogel material, a macromolecule, a polymer, a ceramic particle, a metallic particle, a metal flake, a glass sphere, and a carbon fibre. Furthermore, the step of providing the particles may comprise providing the at least two different types of particles simultaneously (e.g. by a mixture of the at least two different types of particles) or subsequently (e.g. first providing particles of type A and after that providing particles of type B). In this context, irrespective of the fact how the at least two different types of particles are provided, the step of assembling the particles may comprise: First assembling particles of one type of the at least two different types of particles and subsequently, assembling particles of another type of the at least two different types of particles. In case of a mixture of the at least two different types of particles, this can, for example, be realized by using different frequencies for assembling the different types of particles. Advantageously, this provides a simple and reliable way for generating multiparticular (e.g. multicellular) agglomerates.

According to a further aspect, the at least two different types of particles may comprise at least two different biological cell lines. Preferably, one of the at least two different biological cell lines may be larger than another of the at least two different biological cell lines.

According to a further aspect, the one of the at least two different types of particles may be a core material (e.g. a metal flake) encapsulated in hydrogel or a cell line encapsulated in hydrogel. For example, the core material or cell line may be encapsulated in a hydrogel capsule, made from GelMA or Matrigel, which are formed in another process. The encapsulated cell line is preferably much bigger (e.g. 200 µm diameter) than single cells and thus experience a much larger trapping force.

According to a further aspect, the at least two different types of particles may respond differently to acoustic, electric forces and/or magnetic forces. This can be achieved, for example, by including magnetic, gaseous, and/or ionic additives in the hydrogel capsule, mentioned before.

According to a further aspect, the method may further comprise subjecting the assembled agglomerate to a fixation (e.g. via a fixation device). The fixation may comprise a binding (e.g. a solidification and/or connection) of the particles, the working medium, and/or working medium constituents. Advantageously, various types of binding processes are available, which may be selected in dependency on the features of the particles and/or the working medium. If the particles include are, for example, reactive, i.e. capable of providing the binding reaction in their self, the fixation may be obtained simply by maintaining the agglomerate for a certain fixation time, e.g. in a range of seconds to hours. Alternatively, the fixation may be triggered by supplying a fixation input, like at least one of thermal energy input, an irradiation input and a fixation agent input. For applying the thermal energy input, the fixation device may include a thermal source, like e.g. an infrared radiation source, directing thermal energy to the assembling portion. Alternatively, a fixation agent, i.e. a chemical substance, which is capable of providing the binding process between the particles, may be supplied into the working medium. Consequently, the fixation may be preferably a result of biological growth, a timed reaction and/or caused by an external trigger, e.g. thermal energy, irradiation, and/or a fixation agent. Advantageously, the assembled agglomerate can be preserved in its respective shape.

According to a further aspect, the step of providing may comprise providing the particles contained in a working medium in the assembling portion. For example, the working medium may be a hydrogel. Furthermore, the step of subjecting may comprise binding the particles of the assembled agglomerate with the working medium (e.g. by gelling of the working medium via a gelation reaction). If needed, the agglomerate can again be released by an enzymatic degradation of the (fixated) working medium.

According to a further aspect, the step of emitting comprises introducing a frequency-shift and/or phase-shift between the first acoustic holographic interference field and the second acoustic holographic interference field after a predefined period of time. For this, the frequency of the first acoustic holographic interference field (first acoustic holographic interference field frequency) and/or the frequency of the second acoustic holographic interference field (second acoustic holographic interference field frequency) may be changed (e.g. decreased or increased) after starting emitting the first and second acoustic holographic interference fields. For example, the first acoustic holographic interference field may be emitted at a specific frequency for the predefined period of time and after that the specific frequency may be changed to a different value. The changing may be continuously or discrete. After the change, the first and second acoustic holographic interference fields may have a specific (e.g. static or dynamic) offset between their respective frequencies (= frequency-shift). In addition or alternatively, also the (global) phase of the first acoustic holographic interference field (first acoustic holographic interference field phase) and/or the (global) phase of the second acoustic holographic interference field (second acoustic holographic interference field phase) may be changed (e.g. decreased or increased) after starting emitting the first and second acoustic holographic interference fields. This changing may be continuously or discrete. After the change, the first and second acoustic holographic interference fields may have a specific (e.g. static or dynamic) offset between their respective phases (= phase-shift). The afore-mentioned detuning of the first and second acoustic holographic interference fields advantageously allows for varying the (spatial) position of the pressure nodes within the acoustic pressure field. By this, more compact/closed structures can be build.

Also in the context of the method, it is for the skilled person immediately apparent that some of the afore-mentioned aspects (two different types of particles, subjecting the assembled agglomerate to a fixation, hydrogel etc.) described in connection with the emitting of two acoustic holographic interference fields (first and second acoustic holographic interference fields), may also be realized with only one holographic interference field. Consequently, these aspects are also disclosed and claimable independent of the presence of the further (second) holographic interference field. In detail, the afore-mentioned aspects (technically feasible without the second holographic interference field) are also disclosed (individually or in combination) in connection with a method for assembling particles (e.g. in a working medium) to an agglomerate under the effect of acoustic forces, comprising: emitting at least one acoustic holographic interference field (e.g. along a predefined direction) to an assembling portion to form an acoustic pressure field (at the assembling portion), wherein the acoustic pressure field defines a shape of the agglomerate; providing the particles in the assembling portion; and assembling the particles to the agglomerate in the assembling portion via the acoustic pressure field. Preferably, this method may be performed by using an apparatus described herein.

Further details and advantages of the disclosure are described in the following with reference to the attached drawings, which show in:
- Figure 1-9:: schematic illustrations of different embodiments of an apparatus for assembling particles to an agglomerate under the effect of acoustic forces;
- Figure 10:: a diagram of a computed acoustic radiation force of a travelling sound-wave on a particle plotted against the gravitational force;
- Figure 11A-11C:: schematic illustrations of different embodiments of a sample container with a resonance element;
- Figure 12:: a flowchart illustrating of a methods for assembling particles to an agglomerate under the effect of acoustic forces according to an embodiment; and
- Figure 13:: a schematic illustration of a multicellular assembly by using hydrogel capsules according to an embodiment.

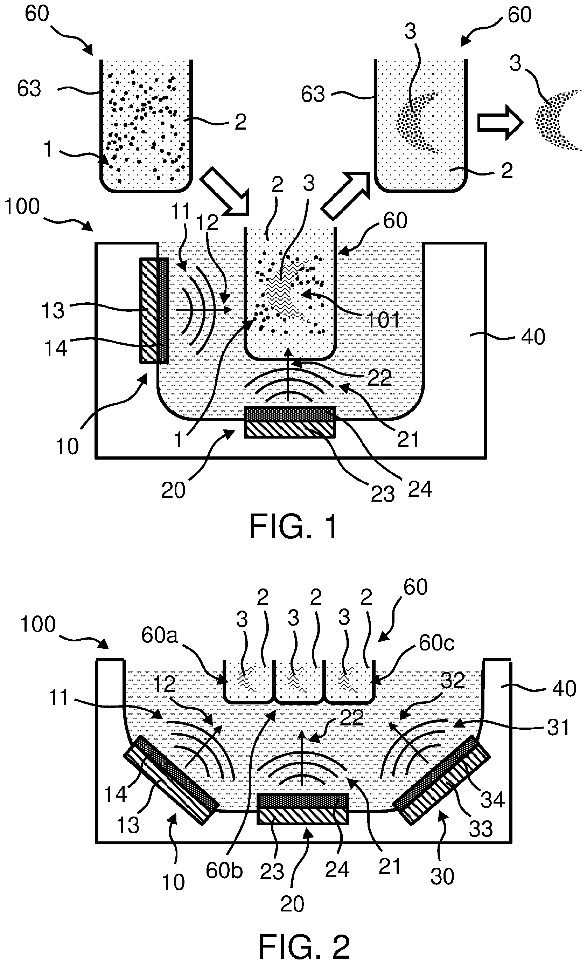

Figures 1-9 each show a schematic illustration of an embodiment of an apparatus 100 for assembling particles 1 to an agglomerate 3 under the effect of acoustic forces.

The particles 1 used in this context may be organic (e.g. comprising bacteria, tissue samples, hydrogels, and/or polymers), inorganic (e.g. comprising polystyrene, glass, ceramics and/or metal), or any combination thereof. Further, the particles 1 may be contained (e.g. suspended) in a, preferably liquid, working medium 2 (e.g. water or a hydrogel). The particles 1 and the working medium 2 may, for example, form a suspension.

The apparatus 100 (e.g. a bio assembler) comprises a first acoustic source device 10 configured for emitting a first acoustic holographic interference field 11 along a first direction 12. Further, the apparatus 100 may comprise a second acoustic source device 20 configured for emitting a second acoustic holographic interference field 21 along a second direction 22. In addition, the apparatus 100 may optionally comprise at least one further acoustic source device 30 configured for emitting at least one further acoustic holographic interference field 31 along at least one further direction 32. A preferred number of acoustic source devices is three or four. For example, as shown in Figures 2, 3, 4, 5, and 8 the at least one further acoustic source device 30 may be a (single) third acoustic source device configured for emitting a third acoustic holographic interference field along a third direction. The acoustic source device 10, 20, 30 may each be configured for emitting acoustic waves with frequencies in a non-audible frequency range, in particular ultrasound frequencies. Particularly preferred, the acoustic waves may have a frequency of at least 20 kHz, in particular at least 40 kHz, up to the GHz-range. Preferably, the acoustic source device 10, 20, 30 may be configured identical and/or identical devices (which differ only in their orientation). The acoustic source device 10, 20, 30 may be configured for (synchronously) emitting acoustic waves of the same frequency.

The first acoustic source device 10 and the second acoustic source device 20 may be arranged such that the first direction 12 and second direction 22 are tilted (e.g. inclined) relative to each other. For example, the first and second direction 12 and 22 may oblique to each other, for example oriented at an obtuse angle to each other. Preferably, the first direction 12 and second direction 22 may be perpendicular to each other. In addition, the at least one further acoustic source device 30 may be arranged such that the at least one further direction 32 is also tilted with respect to the first and/or second direction 12, 22. Preferably, all directions may be (pairwise) oriented obliquely to each other. Each of the directions, i.e. the first, second, and at least one further direction 12, 22, 32, may further be a main emission direction of the respective acoustic source device 10, 20, 30. The (main) direction of each of the acoustic source device 10, 20, 30 may be orientated along their respective axis of maximum radiation intensity. The directions may each be orientated perpendicular to an emission surface (e.g. the middle of the emission surface) of the respective acoustic source device 10, 20, 30.

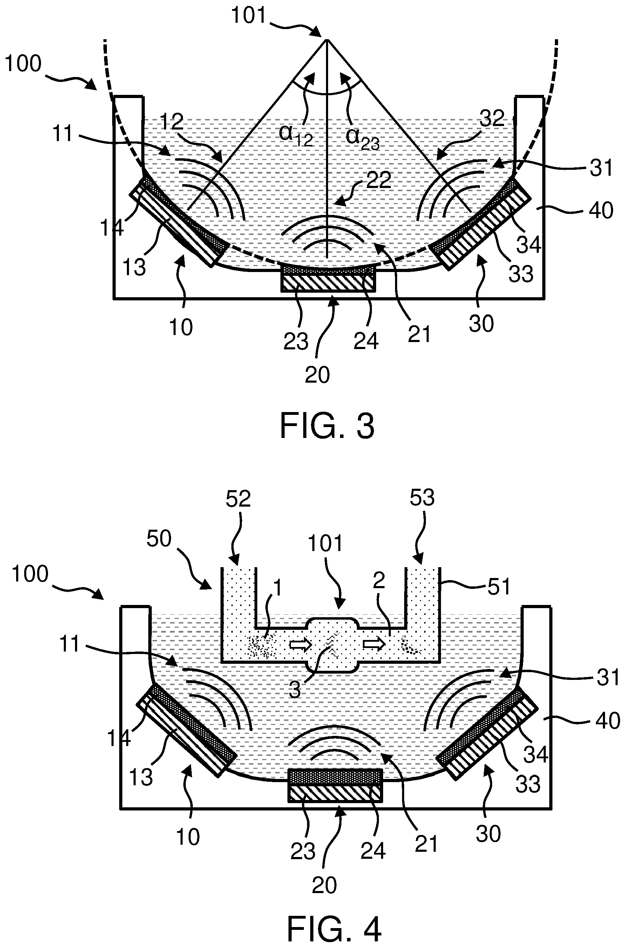

The first acoustic source device 10 and the second acoustic source device 20 may be arranged such that the first acoustic holographic interference field 11 and the second acoustic holographic interference field 21 are superimposed with each other in an assembling portion 101 (of the apparatus 100) to form an acoustic pressure field (at the assembling portion 101). Further, the at least one further acoustic source device 30 may be arranged such that the at least one further acoustic holographic interference field 31 may be superimposed with the first and second acoustic holographic interference fields 11 and 21 in the assembling portion 101 for further forming the acoustic pressure field. In the assembling portion 101 interference of the first, second and/or at least one further acoustic holographic interference field 11, 21, 31 may occur. Consequently, the respective acoustic holographic interference fields 11, 21, 31 may intersect with each other in a common space portion (= the assembling portion 101). Accordingly, the respective directions 12, 22, 32 may intersect with each other in this common space portion (= the assembling portion 101). With respect to this intersection point/portion, the respective tilt angels (e.g. α₁₂ or α₂₃) may be defined (see Figure 3). The respective acoustic source device 10, 20, 30 may be arranged such that they insonify the assembling portion 101 from different/multiple angles.

In this context, the acoustic source devices 10, 20, 30 may be arranged on a (imaginary) hemisphere around the assembling portion 101. The assembling portion 101 may be located in a centre of the (imaginary) hemisphere. Preferably, the assembling portion 101 may be arranged above the acoustic source devices 10, 20, 30 (e.g. in a top region of the apparatus 100). In a preferred embodiment, the acoustic source devices 10, 20, 30 may have a curved (e.g. spherical or cylindrical) emitter surface. Preferably, each of the emitter surfaces is arranged to lie on said hemisphere and/or follows the geometry of said hemisphere (see Figure 3).

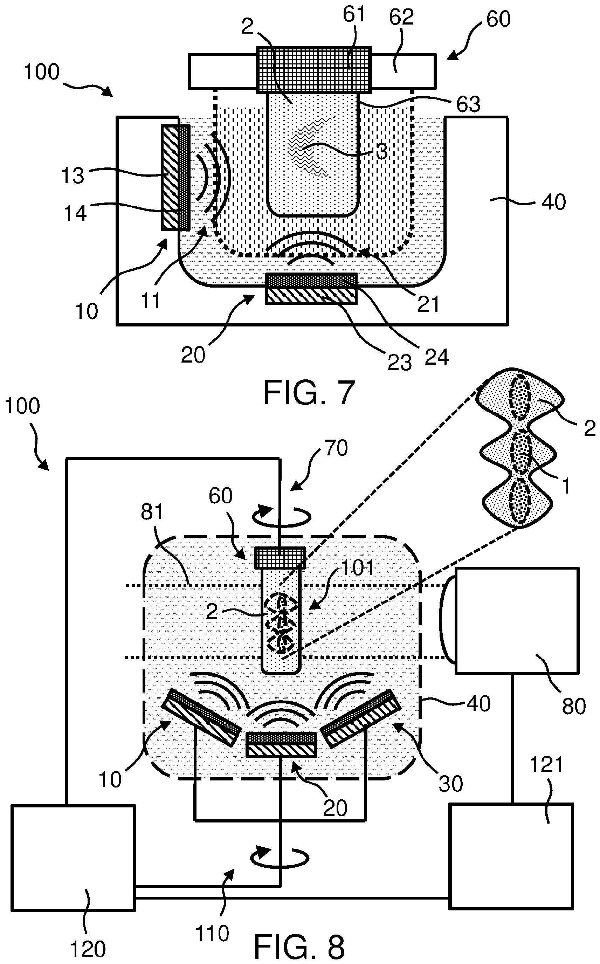

The acoustic source devices 10, 20, 30 may further be rotatable about a common rotation axis. Preferably, this common rotation axis may be orientated vertically. For example, the acoustic source devices 10, 20, 30 may be fixed to a (common) mounting device 110 (see Figure 8), which may be rotatable around the common axis. The mounting device 110 may further be configured to move (translate) the acoustic source devices 10, 20, 30, e.g. up and down.

For generating/emitting the respective acoustic holographic interference fields 11, 21, 31, each of the acoustic source devices 10, 20, 30 may comprise a (e.g. single element) acoustic source 13, 23, 33 (e.g. a transducer, preferably an ultrasound transducer). For example, the first acoustic source device 10 may comprise a first acoustic source 13, the second acoustic source device 20 may comprise a second acoustic source 23, and/or the at least one further acoustic source device 30 may comprise an at least one further acoustic source 33. The acoustic source devices 10, 20, 30 may each comprise a housing, in which the acoustic source 13, 23, 33 is fitted. The respective acoustic sources 13, 23, 33 may have an operating frequency in the range of 0.1 MHz to 100 MHz. The respective acoustic sources 13, 23, 33 may provide high acoustic power output (> 1 W) over long time scales (continuous wave operation). The respective acoustic sources 13, 23, 33 may be water-cooled or air-cooled for optimal performance. In addition or alternatively, the respective acoustic sources 13, 23, 33 may be operated in a pulsed and/or sequential operation. Each of the acoustic source devices 10, 20, 30 may further comprise an acoustic diffractive element 14, 24, 34. For example, the first acoustic source device 10 may comprise a first acoustic diffractive element 14, the second acoustic source device 20 may comprise a second acoustic diffractive element 24, and/or the at least one further acoustic source device 30 may comprise an at least one further acoustic diffractive element 34. The respective acoustic diffractive element 14, 24, 34 may be a (e.g. static) acoustic hologram (e.g. transmission hologram), a (e.g. dynamic) spatial acoustic modulator (e.g. a microbubble array), and/or an acoustic (e.g. labyrinthine) metamaterial. The acoustic diffractive element 14, 24, 34 may be placed on a face or emitting surface of each acoustic source 13, 23, 33. The acoustic diffractive element 14, 24, 34 may shape the emitted sound waves in a way that they form, when superimposed in the assembling portion 101, an acoustic pressure field in the shape of an agglomerate 3 to be created. In the acoustic pressure field, the particles 1 may rearrange and assemble due to acoustic radiation forces and form the agglomerate 3. The acoustic diffractive element 14, 24, 34 may be removable and/or replaceable. Thereby, the acoustic diffractive element 14, 24, 34 may comprise features (e.g. markings) for identifying the respective acoustic diffractive element 14 and for helping positioning/orientating the acoustic diffractive element 14, 24, 34 at the corresponding acoustic sources 13, 23, 33.

Alternatively, for generating/emitting the respective acoustic holographic interference fields 11, 21, 31, each of the acoustic source devices 10, 20, 30 may comprise a phased array transducer. For example, each phased array transducer may comprise an (e.g. integrated) array of acoustic sources (e.g. piezoelectric oscillators), which are individually controllable and each of which being adapted for emitting an acoustic wave. By individually controlling the respective acoustic sources of the array sub-waves may be emitted, which are superimposed (within the phased array transducer) and interfere with each other for generating the respective acoustic holographic interference field.

Irrespective of the concrete implementation of the acoustic source devices 10, 20, 30, each of the acoustic source devices 10, 20, 30 may be frequency-tuneable and/or configured to emit different/multiple frequencies. Consequently, the acoustic source devices 10, 20, 30 may be configured to switch between different operating states. For example, the particles 1 may first be assembled using a first frequency, e.g. for assembling the rough structure of the agglomerate 3, and then be assembled using a second, preferably higher, frequency, e.g. for providing detailed features to the preassembled agglomerate 3. Controlling the phase of each acoustic source device 10, 20, 30 directly or by slightly detuning their relative frequencies may enable to reposition the trap sites over wavelength scale. Due to the continuous nature of the phase, this further enables trapped particles 1 to be transported over length scales of multiple wavelengths, for example, to migrate the particles into a specific region. This further enables, for example, removing particles 1, which populate regions, where no particles are to be trapped.

The apparatus 100 may further comprise a reservoir 40 designed to contain a liquid (e.g. water). The reservoir 40 may be open at the top. The reservoir 40 may be filled with a water bath. The assembling portion 101 may be arranged in the reservoir 40 and/or water bath. For example, the assembling portion 101 may be located at the top and/or in a top region of the reservoir 40. The acoustic source devices 10, 20, 30 may be arranged at the reservoir 40. The acoustic source devices 10, 20, 30 may be arranged for emitting sound waves into the water bath. For example, the acoustic source devices 10, 20, 30 may be attached to a wall (e.g. a side-wall and/or bottom-wall) of the reservoir 40, preferably facing inside the reservoir 40.

The apparatus 100 may further comprise a reservoir insert 40a designed to contain a further liquid (e.g. a further water bath). The reservoir insert 40a may be at least partially inserted in the reservoir 40 to divide the reservoir 40 into two fluidically separated portions 41, 42. For example, the reservoir insert 40a may be configured as a secondary reservoir. The secondary reservoir or reservoir insert 40a may be smaller than the (main) reservoir 40 and may therefore fit in the reservoir 40. The reservoir insert 40a may be inserted in the reservoir 40 such that the assembling portion 101 is located within the reservoir insert 40a. The reservoir insert 40a may comprise a non-permeable housing. For example, the reservoir insert 40a may be a (e.g. thin-walled) stainless steel container. The reservoir insert 40a may be arranged such that it separates the water bath and/or the acoustic source device 10, 20, 30 from an inside of the reservoir insert 40a and/or the further water bath. The reservoir insert 40a may provide a sterile environment in its inside, e.g. for biological cell culture to take place. Preferably, the reservoir insert 40a is removable from the apparatus 100 and/or the reservoir 40, e.g. for sterilizing/autoclaving the reservoir insert 40a between process runs. The reservoir insert 40a may be closable. For example, the reservoir insert 40a may comprise a movable and/or removable cover (e.g. a lid), by means of which the reservoir insert 40a may be closed. In addition or alternatively, the reservoir insert 40a may comprise mounts for fixing a sample container 60 (described in detail below) at the reservoir insert 40a (e.g. at the cover). For example, the reservoir insert 40a may comprise mounts for fixing the sample container 60 inside the reservoir insert 40a. By this, the sample container 60 may be transported within the reservoir insert 40a to or from the apparatus 100. This may enable providing the ability to move the sample e.g. to other laboratories in a sterile environment. Further, the reservoir insert 40a may comprise manipulation means (i.e. a mobile glove box) and/or a manipulation port made of an elastomeric material for an insertion of needles.

The apparatus 100 may further comprise a sample container 60 to contain the particles 1 and/or the working medium 2. The sample container 60 may be arranged at the assembling portion 101. The sample container 60 may be at least partially arranged in the reservoir 40 and/or liquid (e.g. the water bath). Consequently, the sample container 60 may be acoustically coupled to the acoustic source devices 10, 20, 30 via the liquid (e.g. the water bath). The sample container 60 may comprise a reaction tube, a culture plate, a sample tube, a cuvette, a well plate, and/or an insert.

The sample container 60 may have a wall with a thickness sufficiently thin and/or matched with the acoustic wavelength (e.g. a multiple of half the wavelength) to appear sound-transparent. Preferably, the sample container 60 is made of plastics (e.g. polystyrene and/or polypropylene).

The sample container 60 may comprise multiple compartments 60a, 60b, 60c (see Figure 2). For example, the sample container 60 may comprise a well plate with multiple compartments 60a, 60b, 60c. The compartments 60a, 60b, 60c may be separates from each other or connected (e.g. in pairs) via at least one fluid connection and/or semi-permeable membrane. By this, the several agglomerates 3 can be assembled in parallel by emitting the respective acoustic holographic interference fields 11, 21, 31 simultaneously in all compartments 60a, 60b, 60c, or sequentially (e.g. by emitting the respective acoustic holographic interference fields 11, 21, 31 in only one of the compartments 60a, 60b, 60c and moving the compartments 60a, 60b, 60c after each assembling process).

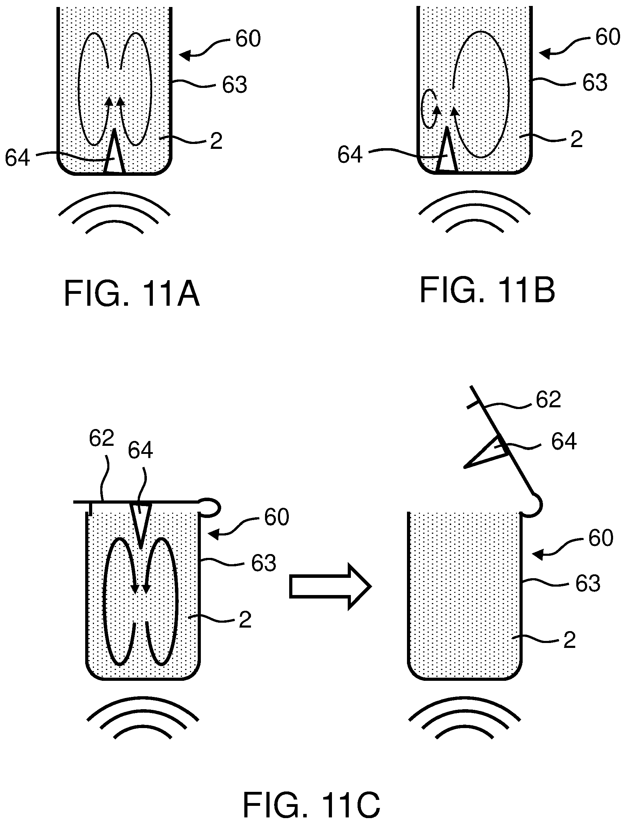

The sample container 60 may further comprise a (e.g. preferably needle-like and/or beam-like) resonance element 64 (see Figures 11A, 11B and 11C). The resonance element 64 may be configured to vibrate when exited by soundwaves (e.g. 100 kHz). The resonance element 64 may be used to generate acoustic streaming flows within the sample container 60 and/or working medium 2 when excited by soundwaves. This may enable quick and efficient mixing of different working media 2 (e.g. two liquids) or re-suspension of the sedimented particles 1 without direct access. The sample container 60 may stay closed and sealed during mixing, an advantage for sterile operation. Acoustic streaming flows may further be used to remove any material (e.g. particles and/or working medium 2) that is not held in the acoustic pressure field. The resonance element 64 may be positioned at a bottom of the sample container 60. Alternatively, the resonance element 64 may be mounted to a lid of the sample container 60, e.g. such that the resonance element 64 is removable from the working medium 2 when the lid is opened. The sample container 60 may comprise at least one optically transparent wall 63. Further, the sample container 60 may comprise a moveable and/or removable cover 62 (e.g. a lid). The sample container 60 may comprise a manipulation port 61 made of an elastomeric material for an insertion of needles. The sample container 60 may comprise a manipulation means (i.e. a mobile glove box). Further, the sample container 60 may comprise a first and secondary casing, wherein the first and secondary casing enclose a portion for holding a sterile liquid.

The apparatus 100 may further comprise a sample container holding device 70 (see Figure 8). The sample container holding device 70 may be configured for fixing the sample container 60 in a predefined position (e.g. at the assembling portion 101) and/or for rotating the sample container 60 about a predefined rotation axis. The predefined rotation axis may, for example, be vertical. The sample container holding device 70 may further be configured to move (translate) the sample container 60, e.g. up and down.

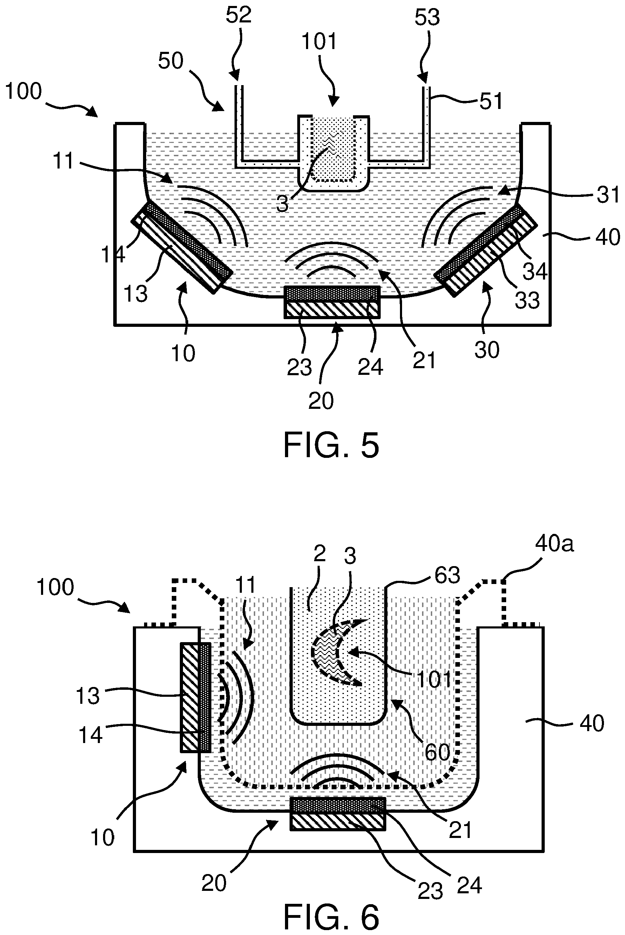

The apparatus 100 may further comprise a flow-through chamber 50 for guiding the particles 1 and/or the working medium 2 (see Figure 4 or 5). The flow-through chamber 50 may comprise a housing 51 with an inlet 52, an outlet 53, and a connection segment 54, which connects the inlet 52 and the outlet 52. The connection segment 54 may be configured for directing a flow of the particles 1 and/or the working medium 2 from the inlet 52 to the outlet 53. The flow-through chamber 50 may be configured for allowing for an exchange of working medium 2 and/or particles 1 via the inlet 52 and outlet 53. Further, the flow-through chamber 50 may be configured for maintaining a predefined CO₂ concentration within the connection segment 54 over longer experiment duration. Thereby, the assembling portion 101 may be arranged within the connection segment 54. Consequently, assembling can be performed inside the connection segment 54 of the flow-through chamber 50. The particles may be assembled by superimposing the acoustic holographic interference fields 11, 21, 31 inside the connection segment 54 to form there the acoustic pressure field in the shape of the desired agglomerate 3. After fixation (e.g. using a light trigger as photo-initiator and/or by waiting a sufficient time, e.g. in the case of biological cells) the fixed agglomerate 3 may be moved with the flow of the particles 1 and/or the working medium 2 to the outlet 53.

In an embodiment, the connection segment 54 of the flow-through chamber 50 may comprise a (e.g. semi-permeable) membrane 55. Preferably, the membrane 55 is configured for allowing for a nutrient exchange through the membrane. The membrane 55 may be arranged to separate the assembling portion 101 from the flow of the particles 1 and/or the working medium 2. Thus, the membrane 55 may be arranged such that it decouples the flow of the particles 1 and/or the working medium 2 from a portion, where the assembling may be performed (= assembling portion 101). Further, the membrane 55 may be arranged to form a flow-calmed portion 56 in the flow-through chamber 50. For example, the membrane 55 may be arranged to divide the interior of the connection segment 54 into at least two regions, wherein one of these regions forms the flow-calmed portion 56 and the other region directs the flow of the particles 1 and/or the working medium 2 from the inlet 52 to the outlet 53. The membrane 55 may form an indentation into the connection segment 54. For example, the connection segment 54 of the housing 51 may comprise an opening, to which the membrane 55 (e.g. membrane bag) is fixed and protrudes from the opening into the interior of the connection segment 54 (e.g. in a bag-like manner). The flow-calmed portion 56 may be open to the outside of the connection segment 54. The membrane 55 may be arranged such that a working liquid and/or the sample container 60 may be inserted in the flow-calmed portion 56. In an embodiment, the membrane 55 forms part of a well plate insert, which is inserted in the connection segment 54. Further, the connection segment 54 may comprise multiple membranes 55 each forming a respective flow-calmed portion 56. By this, multiple (e.g. parallel) separate assembly compartments may be implemented at the connection segment 54, which can be supplied via their own or shared nutrient channels.

The apparatus 100 may further comprise a light projecting device 80. The light projecting device 80 may be configured for illuminating the assembling portion 101 with light 81 (e.g. visible light or ultraviolet light). For example, the light projecting device 80 may comprise a digital micro mirror device and/or a spatial light modulator device. The light projecting device 80 may be configured for creating arbitrary light intensities in the assembling portion 101, e.g. for triggering a chemical reaction there. For enabling tomographic reconstruction of 3D shapes, the light projecting device 80 may be synchronized with said mounting device 110 for rotating the acoustic source devices 10, 20, 30 and/or with said sample container holding device 70 for rotating the sample container 60. For this, the apparatus 100 may further comprise a controller device 120, which may, for example, including a synchronization module 121. The controller device 120 may be configured to (simultaneously) control the movement/rotation of the mounting device 110, the sample container holding device 70, and/or the light emission of the light projecting device 80. The controller device 120 may be configured to control the sample container holding device 70 to rotate the sample container 60 around the predefined rotation axis and/or to control the mounting device 110 to rotate the acoustic source devices 10, 20, 30 around their common rotation axis. The predefined rotation axis of the sample container 60 may be different from the common rotation axis of the acoustic source devices 10, 20, 30 to provide more degrees of freedom for assembling the particles, e.g. during optical curing via the light projecting device 80. A possible product may be formed via a combination of assembling the particles 1 (e.g. cells) under the effect of acoustic forces while shaping the surrounding working medium 2 via fixating under the effect of irradiation is shown in the enlarged portion of Figure 8. It can be seen that the assembled particles 1 (e.g. cells) are shaped (via the acoustic pressure field) in one way, e.g. vertically, and the surrounding working medium 2 (e.g. a gel matrix) is cured (via illumination) in a different way, e.g. horizontally.

The apparatus 100 may further comprise a sedimentation-prevention acoustic source device 90. For example, the sedimentation-prevention acoustic source device 90 may comprise an unfocussed ultrasound transducer. The sedimentation-prevention acoustic source device 90 may be arranged at the bottom of the apparatus 100, e.g. at the bottom of the reservoir 40. The sedimentation-prevention acoustic source device 90 may face upwards and/or may be configured for emitting soundwaves directed opposite to the direction of gravity. The sedimentation-prevention acoustic source device 90 may emit a (levitation) wavelength/frequency different from the wavelength/frequency emitted by the acoustic source devices 10, 20, 30 to assemble the particles 1. By this, a levitation force to the already assembled agglomerate 3 may be provided, while allowing (single) particles 1 to sediment unhindered through the assembling portion 101.

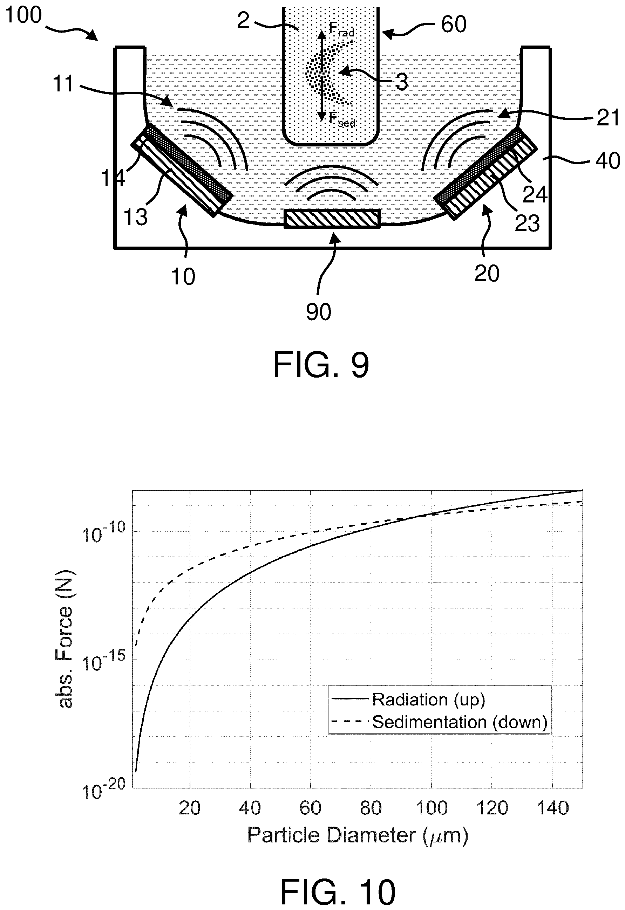

In this context, Figure 10 shows a computed acoustic radiation force of a travelling soundwave (3 MHz, 400 kPa) on NIH-3T3 fibroblast cells plotted against the gravitational force. Agglomerates 3 are assumed to behave like continuous cells in this calculation. The crossover happens around 100 µm diameter. Thus, an unfocused wave of 3 MHz, 400 kPa can be used to prevent sedimentation of larger aggregates without affecting single cells. The acoustic assembling may then be performed at a higher frequency (e.g. 10 MHz). The process can further be equipped with a camera and feedback loop to automatically control the optimal pressure amplitude of the sedimentation-prevention acoustic source device 90 to prevent sedimentation of the assembled agglomerate 3. The sedimentation-prevention acoustic source device 90 may further be weakly focused as long as it appears as a plane wave relative to the overall dimensions of the assembling portion 101.

Therefore, the sedimentation-prevention acoustic source device 90 may emit a low frequency wave (e.g. hundreds of kHz) for providing levitation to prevent the agglomerate 3 from collapsing (see Figure 9). The frequency may also be continuously lowered while an agglomerate 3 forms to always provide the optimal levitation force to counteract sedimentation (e.g. possible with broadband transducers). The levitation wave would typically be an unfocused or weakly focused wave.

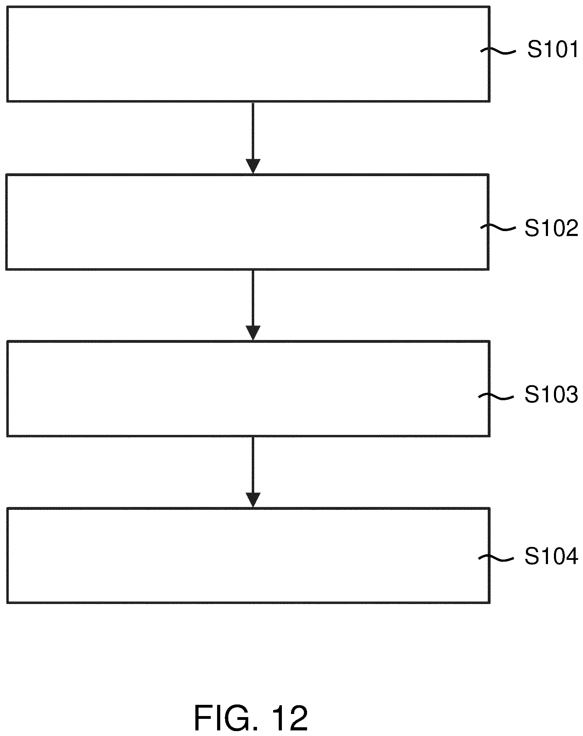

Figure 12 shows a flowchart illustrating of a method for assembling particles 1 to an agglomerate 3 under the effect of acoustic forces according to an embodiment. Preferably, the method is performed by using an apparatus 100 described herein. Further, the particles 1 may be organic (e.g. comprising bacteria, tissue samples, hydrogels, and/or polymers), inorganic (e.g. comprising polystyrene, glass, ceramics and/or metal), or any combination thereof. Further, the particles 1 may be contained (e.g. suspended) in a working medium 2 (e.g. water or a hydrogel).

In step S101 a first acoustic holographic interference field 11 is emitted (e.g. by a first acoustic source device 10) along a first direction 12 and a second acoustic holographic interference field 21 is emitted (e.g. by a second acoustic source device 20) along a second direction 22. Preferably, the first and second acoustic holographic interference fields 11 and 12 are emitted synchronously and/or with the same frequency (e.g. a frequency in a non-audible frequency range, like an ultrasound frequency, for example). Optionally, the method comprises a step of emitting at least one further acoustic holographic interference field 31 (e.g. by at least one further acoustic source device 30) along at least one further direction 32. A preferred number of acoustic holographic interference fields is three or four. For example, the at least one further acoustic holographic interference field 31 may be a (single) third acoustic holographic interference field emitted (e.g. by a third acoustic source device) along a third direction. The first acoustic holographic interference field 11 and the second acoustic holographic interference field 21 are arranged such that the first direction 12 and second direction 22 are tilted relative to each other. For example, the first and second direction 12 and 22 may oblique to each other, for example oriented at an obtuse angle to each other. Preferably, the first direction 12 and second direction 22 may be perpendicular to each other. In addition, the at least one further acoustic holographic interference field 31 may be emitted such that the at least one further direction 32 is also tilted with respect to the first and/or second direction 12, 22. Preferably, all directions may be (pairwise) oriented obliquely to each other.

In step S102, the first acoustic holographic interference field 11 and the second acoustic holographic interference field 12 are superimposed with each other in an assembling portion 101 to form a, preferably stationary, acoustic pressure field (at the assembling portion 101). Further, the at least one further acoustic holographic interference field 31 may be emitted such that the at least one further acoustic holographic interference field 31 may be superimposed with the first and second acoustic holographic interference fields 11 and 21 in the assembling portion 101 for further forming the acoustic pressure field. In the assembling portion 101, the first, second and/or at least one further acoustic holographic interference field 11, 21, 31 may interfere with each other (to form the acoustic pressure field).

In step S103, the particles 1 are provided in the assembling portion 101. This can, for example, comprise positioning said sample container 60 comprising the particles 1 and/or the working medium 2 at the assembling portion 101. In addition or alternatively, the particles 1 may, for example, be transported to the assembling portion 101 via said flow-through chamber 50.

In step S104, the particles 1 are assembled to the agglomerate 3 in the assembling portion 101 via the acoustic pressure field. This may involve moving the particles 1 by the effect of acoustic forces of the acoustic pressure field.

After the agglomerate 3 is assembled, the method may optionally comprise a step of subjecting the assembled agglomerate 3 to a fixation. For example, the fixation may be a result of biological growth, a timed reaction and/or caused by an external trigger, e.g. thermal energy, irradiation, and/or a fixation agent. For example, in the case that the particles 1 are cells the acoustic pressure field may be provided for as long as necessary to allow the cells to attach to each other, thereby fixating the assembled cell agglomerate 3.

In a preferred embodiment, the working medium 2 may be a hydrogel. The hydrogel may be solidified due to a gelation reaction. In the case that the particles 1 are cells, hydrogels may further support the cells and allow further growth in an incubator. Moreover, the assembled agglomerate 3 may, if needed, be released by enzymatic degradation of the hydrogel. The hydrogel may be chosen to gel within a specified time window (e.g. between 1 and 60 min). This allows enough time to remove untrapped particles 1 (e.g. cells) from the assembling portion 101 (e.g. via cross flow, sedimentation, magnetic fields or other forces). Alternatively, the hydrogel may be crosslinked using UV-radiation or temperature. Some hydrogels are capable of reversible transition between liquid and gel state. For example, copolymers of PNIPAam and PEG are liquid between 0 and 15 °C and solidify above 25 °C (e.g. "Mebiol" by Advanced BioMatrix Inc.). This enables a stepwise process where the hydrogel may be solid during cell culture (growth phase at 37 °C) and liquid when new cells need to be added/assembled (process phase at 10 °C). Using thin scaffolding structures (fabric or wire, acoustically transparent) one may support already grown cells (agglomerate 3) so that they do not disintegrate during process phase. This may allow for a stepwise fabrication of multi-cellular organoids. Alternatively, the acoustic pressure field may be used for assembling solid particles in a shape to be desired and beset up in a way to not affect the cells directly (e.g. by varying frequency and acoustic power). The particles 1 can then form a scaffold for cells to attach to.

Multicellular assemblies may also be achieved by sequentially adding different cell types to the apparatus 100, i.e. least two different types of particles 1a and 1b. In one embodiment, the acoustic pressure field properties are chosen to trap single cells. After adding the cell suspension, the cells (= one type of particles 1a) will sediment through the assembling portion 101 and be trapped in the acoustic pressure field. Different cell types (= another type of particles 1b) can then be added sequentially. They will form additional layers on top of the previously added cells. This results in layered shell-like products.

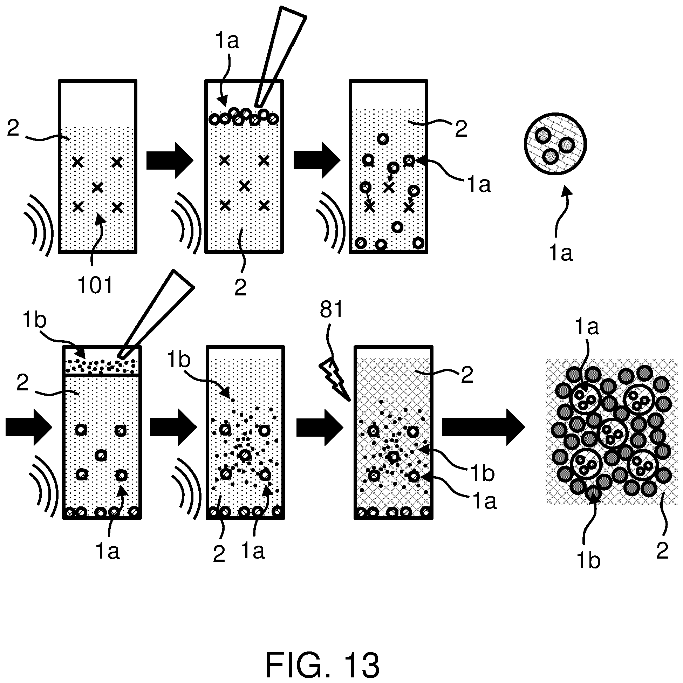

Figure 13 illustrates another embodiment, where the acoustic pressure field is generated to not affect single cells directly. However, one cell-type (e.g. particle type A or one type of particles 1a) may be embedded in hydrogel capsules, e.g. made from GelMA or Matrigel, which may be formed in another process. Reference is made in this context to K. Yue et al. "Synthesis, properties, and biomedical applications of gelatin methcryloyl (GeIMA) hydrogels", Biomaterials 73 (2015), 254-271. The cell-laden capsules may be much bigger (e.g. 200 µm diameter) than single cells (e.g. particle type B or another type of particles 1b) and thus experience a much larger trapping force. The capsules may be added first to the assembling portion 101, where they may be held back by the acoustic radiation force in the acoustic pressure field. The single cell types are then added and sediment through the assembling portion 101. Because single cells do not experience a sufficient acoustic trapping force, in this embodiment, they may be distributed more evenly through the volume. Finally, the whole volume may be fixed (e.g. with the help of a trigger, such as heat, light 81, and/or a timed reaction), and the product can be cut out as a section. Using this process, one cell type can be spatially structured within another cell type to form a functional tissue. Multicellular assemblies can further be realized by combining different transport technologies (e.g. acoustics and magnetics).

For example, a first cell line (e.g. particle type A or one type of particles 1a) may have magnetic nanoparticles incorporated either directly or in combination within a hydrogel capsule. During acoustic assembly (acoustic field is on) untrapped cells may be swept away by a magnetic field gradient. To add a second different cell line (e.g. particle type B or another type of particles 1b), the acoustic field may be turned off and the magnetic field gradient adjusted in a way to prevent sedimentation of the assembled cells. The second cell line may pass through the assembling portion 101 due to sedimentation. Now, the acoustic field may be turned back on using the same or a different hologram, i.e. projecting the same or a different pattern. The process may last until the remaining untrapped cells passed the assembling portion 101. Then the whole phase may be solidified using UV, heat or by adding a cross-linker.

A similar multicell process works in reverse order, where the first cell line (one type of particles 1a) may be non-magnetic and may settle under gravity. The agglomerate 3 is formed as previously described while the acoustic pressure field may be on. To add a second different cell line (another type of particles 1b), total density and viscosity of the suspension may be increased by adding sugar or changing the temperature. This may prevent further sedimentation of the cells under gravity. The second cell line may be responsive to magnetism by incorporating nanoparticles. Then the acoustic field may be turned off and the magnetic field gradient may adjust in a way to transport the second cell line through the assembling portion 101. Now the acoustic field may be turned back on using the same or a different hologram, i.e. projecting the same or a different pattern. The process lasts until the remaining untrapped, magnetic cells passed the assembling portion 101. Then the whole phase may be fixated, e.g. using UV, heat or by adding a cross-linker.

Another embodiment may use light exposure to instantaneously fix cell positions in 3D. In this process, the cells may pass through a coarse hydrogel (e.g. via sedimentation or cross-flow). Cells and hydrogel may be both functionalized (e.g. using RGD peptides). Light-based trigger may activate the binding sites in the matrix, so the cells may bind to the closest counterpart in the hydrogel mesh and are thus fixed in place. After, the cell may be suitably attached by incubation, the remaining sites may turned inactive again (e.g. exposure at a different wavelength or functionalization). This process can then be repeated for many different cell types and acoustic pressure field patterns.

Alternatively, besides sedimentation or flow-through systems, one can also directly move the sample container 60 relative to the projected acoustic field. Prior to the process, the particles 1 may be located at the bottom (e.g. after centrifugation) of the sample container 60 filled with working medium 2. During the process, the sample container 60 may be slowly moved from top to bottom through the acoustic pressure field. When passing the trapping sites, particles 1 may be picked up and held in the trap and thus move upward relative to the sample container 60. The sample container 60 may be moved in a way slow enough so that the hydrodynamic drag does not push particles out of the acoustic pressure field. This embodiment may enable acoustic assembly even when working medium 2 and particles 1 are density matched or particle sedimentation would take too much time.

Although the invention has been described with reference to certain exemplary embodiments, it is evident to a person skilled in the art that various changes can be implemented and equivalents can be used as substitute without departing from the scope of the invention. Consequently, the invention is not to be limited to the disclosed exemplary embodiments, but is to comprise all exemplary embodiments falling within the scope of the attached patent claims. More particularly the invention also claims protection for the subject-matter and the features of the dependent claims independently of the referenced claims.

### Reference list

- 1: particle
- 1a: one type of particles
- 1b: another type of particles
- 2: working medium
- 3: agglomerate
- 10: first acoustic source device
- 11: first acoustic holographic interference field
- 12: first direction
- 13: acoustic source
- 14: acoustic diffractive element
- 20: second acoustic source device
- 21: second acoustic holographic interference field
- 22: second direction
- 23: second acoustic source
- 24: second acoustic diffractive element
- 30: at least one further acoustic source device
- 31: at least one further acoustic holographic interference field
- 32: at least one further direction
- 33: at least one further acoustic source
- 34: at least one further acoustic diffractive element
- 40: reservoir
- 40a: reservoir insert
- 50: flow-through chamber
- 51: housing
- 52: inlet
- 53: outlet
- 54: connection segment
- 55: membrane
- 60: sample container
- 60a, 60b, 60c: compartment
- 61: manipulation port
- 62: cover
- 63: wall
- 64: resonance element
- 70: sample container holding device
- 80: light projecting device
- 81: light
- 90: sedimentation-prevention acoustic source device
- 100: apparatus
- 101: assembling portion
- 110: mounting device
- 120: controller device
- 121: synchronization module

## Claims

**1.** Apparatus (100) for assembling particles (1) in a working medium (2) to an agglomerate (3) under the effect of acoustic forces, comprising:
a first acoustic source device (10) configured for emitting a first acoustic holographic interference field (11) along a first direction (12);
a second acoustic source device (20) configured for emitting a second acoustic holographic interference field (21) along a second direction (22);
wherein the first acoustic source device (10) and the second acoustic source device (20) are arranged such that the first acoustic holographic interference field (11) and the second acoustic holographic interference field (21) are superimposed with each other in an assembling portion (101) to form an acoustic pressure field, which defines a shape of the agglomerate (3); and
wherein the first acoustic source device (10) and the second acoustic source device (20) are arranged such that the first direction (12) and second direction (22) are tilted relative to each other.

**2.** Apparatus (100) according to claim 1, wherein:
a) the first direction (12) and the second direction (22) are tilted relative to each other by an angle in a range from 10° to 170°, preferably in a range from 45° to 135°; particular preferred in a range from 80° to 100°; and/or
b) the first direction (12) and the second direction (22) are perpendicular to each other; and/or
c) the first acoustic source device (10) and the second acoustic source device (20) are both arranged on a hemisphere around the assembling portion (101), wherein preferably the assembling portion (101) is located in a centre of the hemisphere; and/or
d) the first acoustic source device (10) and the second acoustic source device (20) do not face each other; and/or
e) the first acoustic source device (10) and the second acoustic source device (20) are rotatable about a common rotation axis.

**3.** Apparatus (100) according to any one of the preceding claims, wherein:
a₁) the first acoustic source device (10) has a curved, preferably spherical or cylindrical, emitter surface; and/or
a₂) the first acoustic source device (10) is a, preferably frequency-tunable, ultrasound source device, preferably configured for emitting sound waves in a frequency range from 100 kHz to 50 MHz; and/or
a₃) the first acoustic source device (10) is a phased array transducer; and/or
a₄) the first acoustic source device (10) comprises a first acoustic source (13), preferably a piezoelectric oscillator, and a first acoustic diffractive element (14), preferably a transmission hologram; and/or
b₁) the second acoustic source device (20) has a curved, preferably spherical or cylindrical, emitter surface; and/or
b₂) the second acoustic source device (20) is a, preferably frequency-tunable, ultrasound source device, preferably configured for emitting sound waves in a frequency range from 100 kHz to 50 MHz; and/or
b₃) the second acoustic source device (20) is a phased array transducer; and/or
b₄) the second acoustic source device (20) comprises a second acoustic source (23), preferably a piezoelectric oscillator, and a second acoustic diffractive element (24), preferably a transmission hologram.

**4.** Apparatus (100) according to any one of the preceding claims, further comprising:
at least one further acoustic source device (30) configured for emitting at least one further acoustic holographic interference field (31) along at least one further direction (32);
wherein the at least one further acoustic source device (30) is arranged such that the at least one further acoustic holographic interference field (31) is superimposed with the first and second acoustic holographic interference fields (11, 21) in the assembling portion (101) for further forming the acoustic pressure field; and
wherein the at least one further acoustic source device (30) is arranged such that the at least one further direction (32) is tilted, preferably perpendicular, with respect to the first direction (12) and/or second direction (22).

**5.** Apparatus (100) according to any one of the preceding claims, further comprising:
a reservoir (40) designed to contain a liquid, preferably water;
wherein the first acoustic source device (10) and the second acoustic source device (20), and preferably the at least one further acoustic source device (30), are arranged at the reservoir (40) and/or are attached to a wall of the reservoir (40).

**6.** Apparatus (100) according to claim 5, further comprising:
a, preferably removable, reservoir insert (40a) designed to contain a further liquid;
wherein the reservoir insert (40a) is at least partially inserted in the reservoir (40) to divide the reservoir (40) into two fluidically separated portions (41, 42).

**7.** Apparatus (100) according to any one of the preceding claims, further comprising:
a flow-through chamber (50) for guiding the particles (1) and/or the working medium (2);
wherein the flow-through chamber (50) comprises a housing (51) with an inlet (52), an outlet (53), and a connection segment (54), which connects the inlet (52) and outlet (52);
wherein the connection segment (54) is configured for directing a flow of the particles (1) and/or the working medium (2) from the inlet (52) to the outlet (53); and
wherein the assembling portion (101) is arranged within the connection segment (54).

**8.** Apparatus (100) according to claim 7, wherein:
the connection segment (54) of the flow-through chamber (50) comprises a, preferably semi-permeable, membrane (55) preferably being arranged for forming a flow-calmed portion (56) in the flow-through chamber (50);
wherein the membrane (55) is arranged to separate the assembling portion (101) from the flow of the particles (1) and/or the working medium (2).

**9.** Apparatus (100) according to any one of the preceding claims, further comprising:
a sample container (60) to contain the particles (1) and/or the working medium (2), wherein the sample container (1) is arranged at the assembling portion (101).

**10.** Apparatus (100) according to claim 9, wherein the sample container (60):
a) comprises multiple compartments (60a, 60b, 60c), preferably connected in pairs via a fluid connection and/or a semi-permeable membrane; and/or
b) comprises a manipulation port (61) made of an elastomeric material for an insertion of needles; and/or
c) comprises a removable cover (62); and/or
d) comprises at least one optically transparent wall (63); and/or
e) comprises a, preferably needle-like and/or beam-like, resonance element (64), configured to vibrate when exited by soundwaves; and/or
f) is made of a soft, dissolvable and/or enzymatically digestible material.

**11.** Apparatus (100) according to claim 9 or 10, further comprising:
a sample container holding device (70) configured for fixing the sample container (60) in a predefined position and/or configured for rotating the sample container (60) about a predefined rotation axis.

**12.** Apparatus (100) according to any one of the preceding claims, further comprising:
a light projecting device (80), preferably comprising a digital micro mirror device and/or a spatial light modulator device, configured for illuminating the assembling portion (101) with light, preferably to locally trigger chemical reactions and/or induce local solidification of the agglomerate (3).

**13.** Apparatus (100) according to any one of the preceding claims, further comprising:
a sedimentation-prevention acoustic source device (90) arranged, preferably at the bottom of the apparatus (100), for emitting soundwaves directed opposite to the direction of gravity, preferably to prevent sedimentation of the particles (1) and/or the agglomerate (3).

**13.** Method for assembling particles (1) in a working medium (2) to an agglomerate (3) under the effect of acoustic forces, preferably by using the apparatus (100) according to one of the preceding claims, comprising:
emitting a first acoustic holographic interference field (11) along a first direction (12) and emitting a second acoustic holographic interference field (21) along a second direction (22);
wherein preferably the first and second acoustic holographic interference fields (11, 21) are emitted with the same frequency and/or synchronized to each other;
wherein the first direction (12) and second direction (22) are tilted relative to each other;
superimposing the first acoustic holographic interference field (11) and the second acoustic holographic interference field (21) with each other in an assembling portion (101) to form an acoustic pressure field, which defines a shape of the agglomerate (3);
providing the particles (1) in the assembling portion (101); and
assembling the particles (1) to the agglomerate (3) in the assembling portion (101) via the acoustic pressure field.

**14.** Method according to claim 13, wherein:
the particles (1) comprise at least two different types of particles (1a, 1b), preferably selected from the following materials: a biological cell, a stem cell, a spheroid, an organoid, a tissue fragment, a biomolecule, a bacterium, a virus, a gel bead, a vesicle, a tissue scaffold material, a hydrogel material, a macromolecule, a polymer, a ceramic particle, a metallic particle, a metal flake, a glass sphere, and a carbon fibre; and
the step of providing the particles (1) comprises providing the least two different types of particles (1a,1b) simultaneously or subsequently.

**15.** Method according to claim 14, wherein:
the at least two different types of particles (1a,1b) comprise at least two different biological cell lines, wherein preferably one of the at least two different biological cell lines is larger than another of the at least two different biological cell lines; and/or
one of the at least two different types of particles (1a,1b) is a cell line encapsulated in hydrogel; and/or
wherein the at least two different types of particles (1a,1b) respond differently to acoustic, electric forces and/or magnetic forces.

**16.** Method according to any one of claims 13 to 15, further comprising:
subjecting the assembled agglomerate (3) to a fixation, wherein preferably the fixation is a result of biological growth, a timed reaction and/or caused by an external trigger, e.g. thermal energy, irradiation, and/or a fixation agent.

**17.** Method according to claim 16, wherein:
the step of providing comprises providing the particles (1) contained in a working medium (2) in the assembling portion (101), wherein preferably the working medium (2) is a hydrogel; and
the step of subjecting comprises binding the particles (1) of the assembled agglomerate (3) with the working medium (2).

**18.** Method according to any one of claims 13 to 17, wherein the step of emitting comprises:
introducing a frequency- and/or phase-shift between the first acoustic holographic interference field (11) and the second acoustic holographic interference field (21) after a predefined period of time.
